# EUROPEAN PATENT APPLICATION

(11) **EP 1 413 328 A1**
(43) Date of publication of application: **28.04.2004**
(21) Application number: 02425636.4
(22) Date of filing: 21.10.2002
(51) Int. Cl.: A61M 39/02, A61M 39/26

(54) **Valve for medical use**

(71) Applicant: Bioservice S.p.A., 46025 Poggio Rusco (MN) (IT)
(72) Inventor: Baraldi, Massimo, 41036 Medolla (MO) (IT); Musicco, Gian Franco, 25124 Brescia (BS) (IT)
(74) Representative: Gislon, Gabriele

(57) **Abstract**

A valve for medical use for drawing or administering fluids in a device (5) for the transport of body fluids comprises a seat (2) in permanent fluid communication with the device (5) and an elastically compressible element (7) housed in the seat (2) and mobile under the action of a cone (13) of a syringe without a needle(6A) from an engaged position of the seat with the closed valve to a partially released position of said seat with the open valve.

## Description

The present invention concerns a valve for medical use and more particularly a valve for drawing or administering fluids in a system for the transport of body fluids, by means of a syringe without a needle.

The term "system for the transport of body fluids" indicates here all the systems for transporting fluids from the outside to the patient (for example with phleboclysis) or from the patient to the outside, for example systems for thoracentesis or catheters for urine, even if they do not involve direct contact with the patient's blood.

The known means for drawing (or administering) fluids in a system for the transport of body fluids comprise a connecting element between two portions of tube and a derivation protruding from said connection provided with a septum of elastomeric material. The septum is perforated by the needle of a syringe when a fluid has to be drawn or administered.

This system has the disadvantage of having to use a needle which, after having been withdrawn from the septum, may accidentally infect the operator. To avoid this risk of infection from an accidental jag, the necessity has been arisen to have valves which substitute the perforatable septum and which allow drawing or administration with only the connecting cone of the syringe in the absence of any needle.

The aim of the present invention is to provide a valve for a connection in a system for the transport of body fluids which may be operated by a syringe without a needle, and which is reliable and economic to be produced.

This aim is achieved by means of the present invention which concerns a valve for medical use in devices for the transport of fluids, characterised in that it comprises:
a seat having a proximal end and a distal end with respect to said fluid transport device, said proximal end being in permanent fluid communication with said device and said distal end being provided with an opening that can be engaged with a syringe without a needle; and
an elastic sealing element housed in said seat, said element being compressible from a closed position where it engages said distal end of said seat to an open position where said distal end is released from said elastic element.

According to a preferred embodiment, the elastic body comprises a distal portion that may be partly engaged as a seal with said distal end of said seat and partly engaged by said syringe, and at least one elastic and axially compressible leg, engaged with said proximal end of said seat.

In the preferred embodiment, the valve is composed of said seat and of said elastic body, and the seat is made up of two pieces bonded together.

The valve according to the invention presents the advantage of being very simple and economic to be prepared, even though it is completely reliable in the fluid seal when it is not used for drawing.

The invention will now be described in greater detail, with reference to the drawings enclosed for illustrative purposes without any limitation, wherein:

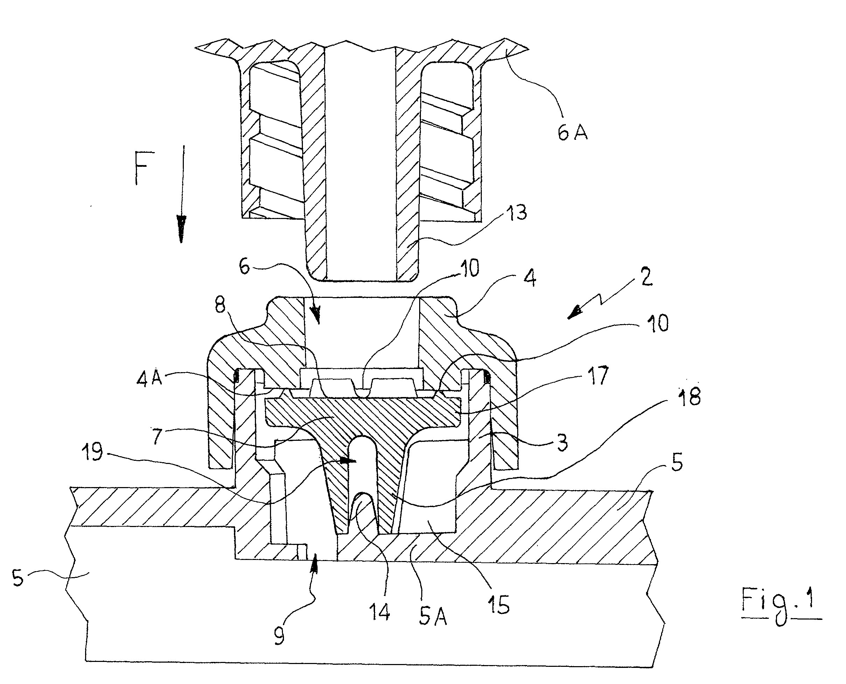

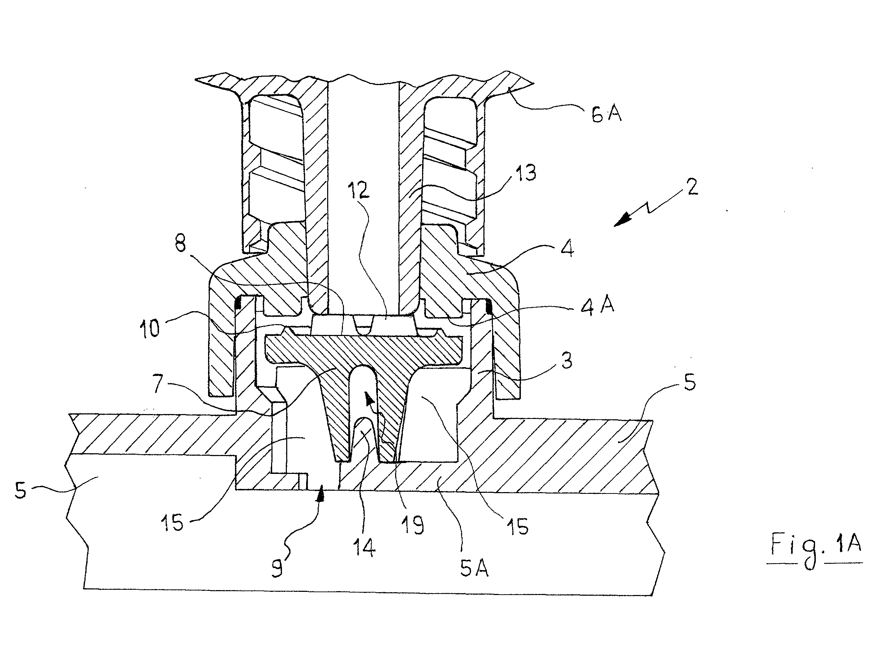

- fig. 1 is a partial cross-section view of the valve according to the invention;
- fig. 1 a is a view similar to the one in fig. 1, in which the elastic element is compressed and the valve is open;
- fig. 2 and fig. 3 are respectively a cross-section and a layout of the elastic element of the valve in fig. 1;

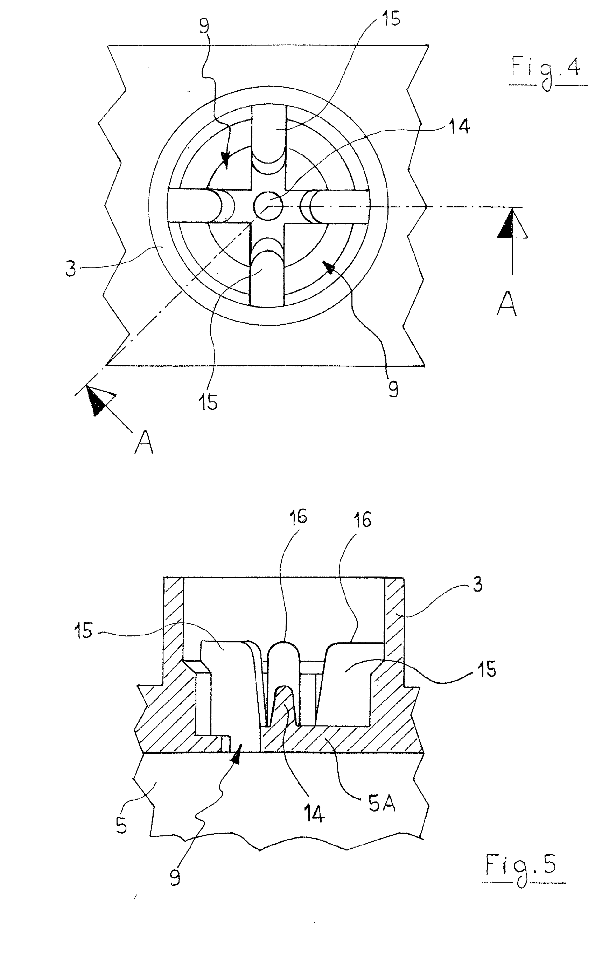

- fig. 4 is a layout of the proximal portion of the seat in fig. 1; and
- fig. 5 is a cross-section along the line A-A of the seat portion in fig. 4.

With reference to figs. 1-3, the valve according to the invention comprises a seat 2 made up of two portions 3 and 4 bonded together, preferably in an irreversible manner. The portion 3 is composed of a derivation of a connector element 5, of a type known in the prior art and used to connect two pipes of a medical device for the transport of fluids. The derivation 3 is all in one piece with said coupling or connector 5.

The portion 4 is composed of a cover welded or glued onto portion 3; the cover 4 corresponds substantially to a distal end of the seat 2 with respect to the device for the transport of fluids which may be represented with 5. Said distal portion 4 of the seat 2 presents an opening 6 which has the function of allowing the fixing cone 13 of the needle of a syringe without a needle 6A, for example a Luer cone or Luer lock, to engage with the top surface 8 of the distal portion of an elastic body 7 housed in the seat 2 (fig. 1 A).

The opposite end of the seat 2, that is the proximal end 5A, is in permanent fluid communication with the fluid transport device, in this case with the connector 5, by means of at least one opening 9.

The elastic element 7 comprises in a single piece a head 17 and a leg 18 with an internal cavity 19. The hollow leg 18 is compressible in a substantially axial direction by the Luer cone (or conical connection) 13 of the syringe without a needle 6A. The top surface 8 of the head 17 of the element 7 presents an annular projection 10 which in rest position (closed valve) engages in a sealed manner with the internal surface 4A of the cover 4. On the surface 8 and inside the annular projection 10 there are a plurality of projections and/or recesses for the passage of the fluid.

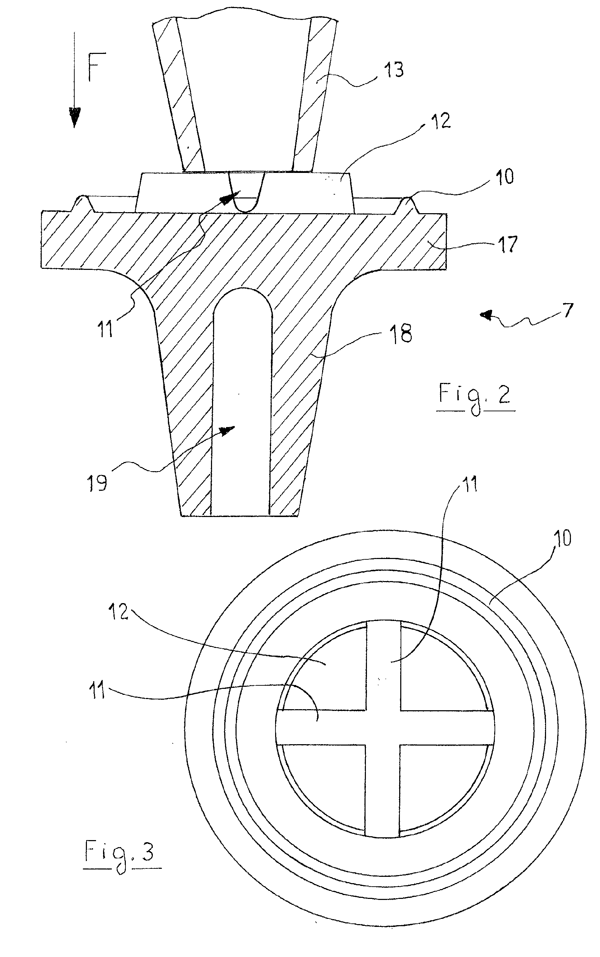

In the embodiment shown, said plurality is obtained by means of two grooves 11 laid crosswise on a circular surface 12 protruding from the surface portion 8 and engaged by the cone 13 during the use of the device. In this way the passage of the fluid is allowed along the grooves 11 even when the surface 12 is engaged by the cone 13 of the syringe 6A (fig. 1 A and fig. 2) during the compression phase of the element 7.

To guarantee the seal, the dimensions of the elastic element 7 and those of the seat 2 are preferably such as to have a reduced compression of the element 7 itself even in rest position, so as to obtain a seal between the ring 10 and the surface 4A. As already said above, the element 7 may be further axially compressed (arrow F) by the syringe cone 13. The elastic element 7 is preferably all in one piece and is made of an elastomeric material, for example latex, neoprene, (poly)isoprene or thermoplastic rubber and has preferably a hardness in the range comprised between 20 and 90 Shore A.

The leg of the element 7 is hollow for easier compression and for better positioning of the same. In fact, in the cavity 19 is housed a guide tooth 14 protruding into the seat 2 from the proximal end 5A of the same, in a central position and adjacent to the opening 9. In addition to the tooth 14 there are provided further means for guiding the leg, composed of a plurality of radial fins 15 extending vertically into the seat 2. The top surface 16 of the fins 15 also has the function of a stop, that is a limit stop, for the compression of the element 7 by the syringe 6A. In one embodiment of the invention the fins are made of elastomeric material on the leg itself of the elastic element 7 and integral with it.

The dimensions of the portion corresponding to the opening 6 are such that its internal diameter has a value slightly smaller than the external diameter of the cone 13, so as to obtain a substantial adherence between the cone and the walls of the cover when the cone 13 is inserted into the opening 6. In turn, the height of the opening 6 is such as to allow the cone 13 to penetrate inside the opening 6 enough to be able to crush the element 7 to the bottom of its stroke against the fins 15. Since the dimensions of the cones 13 of syringes are standardised, the creation of an opening 6 corresponding to the requirements described above is simple and immediate.

The operation of the valve according to the invention is extremely simple. When the cone 13 engages with the surface 8 of the element 7 at the projections 12 and axially compresses said element according to the arrow F, the protruding annular seal 10 is released from the internal surface 4A of the cover 4 and the head of the element 7 is lowered, generally until it touches the surface 16 of the fins 15. Since the diameter of the head 17 of the element 7 is smaller than the diameter and than the dimensions of the corresponding portion of the seat 2, a continuous connection is formed between the syringe cone 13 and the connector 5 of the fluid transport device, said connection comprising the grooves 11, the space between the surface 8 and the internal wall 4A of the cover 4, the space at the side between the head 17 of the elastic element 7 and the seat 2 and the space between the fins 15 including the openings 9 on the proximal end of the seat 2.

During drawing, a seal, against the escaping of the fluid, is guaranteed by the substantial adherence between the cone 13 and the internal walls of the cover 4 and by the low pressure value inside the connector 5.

Once the fluid has been drawn or administered, after withdrawal of the cone 13 from the opening 16, the elastic element will again assume its initial position, shown in fig. 1, guaranteeing the valve seal.

## Claims

1. Valve for medical use in devices for the transport of fluids, **characterised in that** it comprises:
a seat (2) having a proximal end and a distal end (4, 4A) with respect to said fluid transport device, said proximal end being in permanent fluid communication (9) with said device and said distal end being provided with an opening (6) that can be engaged with a syringe (6A) without a needle; and
an elastic sealing element (7) housed in said seat (2), said element being compressible from a closed position where it is engaged with said distal end (4, 4A) of said seat to an open position where said distal end is released from said elastic element.

2. Valve according to claim 1, **characterised in that** said elastic element comprises:
a distal portion (17) that may be partly engaged as a seal (10) with said distal end (4, 4A) of said seat and partly engaged (12) by said syringe, and at least one elastic and axially compressible leg (18) engaged with said proximal end (5A) of said seat.

3. Valve according to claim 2, **characterised in that** said seat and/or said elastic element (7) present means (14, 15) for guiding the axial compression of said leg (18).

4. Valve according to claim 2 or 3, **characterised in that** the surface (8) of said distal portion (17) in said elastic element (7) presents a plurality of projections (12) and/or grooves (11) to allow a passage of fluid when said elastic element is engaged and compressed by the cone (13) of said syringe (6A).

5. Valve according to one of the previous claims, **characterised in that** said elastic element (7) is made in a single piece from a material with hardness in the range comprised between 20 and 90 Shore A.

6. Valve according to one of the claims from 2 to 5, **characterised in that** said leg (18) presents a cavity (19) in which a guide tooth (14) is housed.

7. Valve according to one of the previous claims, **characterised in that** said seat is made up of two portions (3, 4) bonded together and **in that** one of them (4) is in a single piece with a pipe connecting element (5).

8. Device for medical use for the transport of fluids, comprising a valve according to one of the previous claims.

9. Pipe connecting element (5) for medical use, comprising a valve according to one of the claims from 1 to 7.
